(19) Europäisches Patentamt
European Patent Office
Office européen des brevets

(11) **EP 3 811 032 B1**

(12) **FASCICULE DE BREVET EUROPEEN**

(45) Date de publication et mention
de la délivrance du brevet:
**28.01.2026 Bulletin 2026/05**

(21) Numéro de dépôt: **19731747.2**

(22) Date de dépôt: **21.06.2019**

(51) Classification Internationale des Brevets (IPC):
**G01C 22/00** (2006.01)     **A61B 5/22** (2006.01)
**A61B 5/11** (2006.01)      **A61B 5/00** (2006.01)

(52) Classification Coopérative des Brevets (CPC):
**A61B 5/224; A61B 5/112; A61B 5/6829;**
**G01C 22/006;** A61B 5/6828; A61B 5/6831;
A61B 2562/0219

(86) Numéro de dépôt international:
**PCT/EP2019/066534**

(87) Numéro de publication internationale:
**WO 2019/243609 (26.12.2019 Gazette 2019/52)**

(54) **ANALYSE DE LA FOULEE D'UN PIETON EN MARCHE**

ANALYSE DES GANGS EINES GEHENDEN FUSSGÄNGERS

ANALYSIS OF THE STRIDE OF A WALKING PEDESTRIAN

(84) Etats contractants désignés:
**AL AT BE BG CH CY CZ DE DK EE ES FI FR GB
GR HR HU IE IS IT LI LT LU LV MC MK MT NL NO
PL PT RO RS SE SI SK SM TR**

(30) Priorité: **21.06.2018 FR 1855524**

(43) Date de publication de la demande:
**28.04.2021 Bulletin 2021/17**

(73) Titulaire: **SYSNAV**
**27200 Vernon (FR)**

(72) Inventeurs:
• **VISSIÈRE, David**
  **27200 Vernon (FR)**
• **GRELET, Marc**
  **27200 Vernon (FR)**

(74) Mandataire: **Regimbeau**
**20, rue de Chazelles**
**75847 Paris Cedex 17 (FR)**

(56) Documents cités:
**WO-A1-2014/135187**

• **REN SCHWESIG ET AL: "Inertial sensor based reference gait data for healthy subjects", GAIT & POSTURE, ELSEVIER, AMSTERDAM, NL, vol. 33, no. 4, 25 February 2011 (2011-02-25), pages 673 - 678, XP028480949, ISSN: 0966-6362, [retrieved on 20110302], DOI: 10.1016/ J.GAITPOST.2011.02.023**
• **SEFERIAN A ET AL: "Longitudinal results of magneto-inertial motion analysis in Duchenne muscular dystrophy ambulant patients", NEUROMUSCULAR DISORDERS, vol. 26, 2016, XP029720806, ISSN: 0960-8966, DOI: 10.1016/ J.NMD.2016.06.357**
• **DANIELA TARNITA: "Wearable sensors used for human gait analysis", ROMANIAN JOURNAL OF MORPHOLOGY AND EMBRYOLOGY = REVUE ROUMAINE DE MORPHOLOGIE ET EMBRYOLOGIE, 1 January 2016 (2016-01-01), Romania, pages 373 - 382, XP055562626, Retrieved from the Internet <URL:http://www. rjme.ro/RJME/resources/files/570216373382. pdf> [retrieved on 20190227]**

**Description**

## DOMAINE DE L'INVENTION

La présente invention concerne le domaine de l'analyse de foulée d'un piéton en marche.

## ARRIERE-PLAN TECHNOLOGIQUE

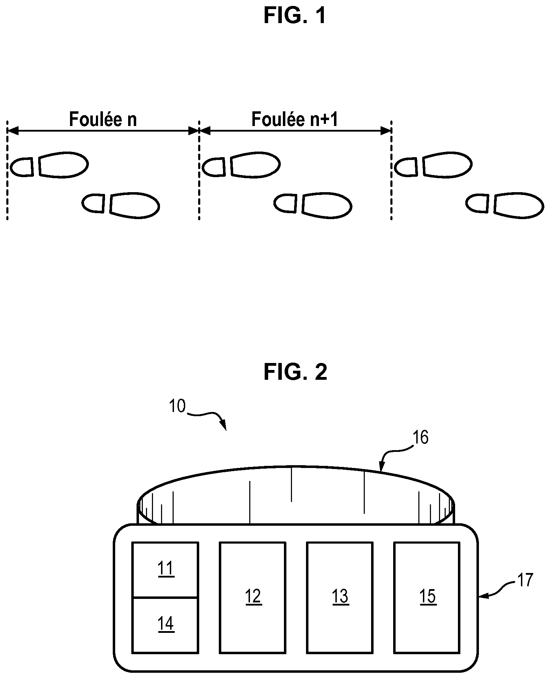

Une marche d'un individu comprend une séquence d'une ou de plusieurs foulées. Une foulée est constituée de deux phases : une première phase pendant laquelle le pied est en contact avec le sol, et une deuxième phase pendant laquelle le pied est en l'air. Ainsi qu'illustré en figure 1, une foulée commence quand un pied touche le sol, et se termine quand le même pied touche à nouveau le sol.

La marche nécessite l'utilisation et la coordination de plusieurs muscles. L'analyse d'une foulée peut donc être utilisée pour caractériser l'état de forme physique d'un individu, et notamment la force musculaire que ce dernier est capable de développer. Cette force musculaire est susceptible de varier en fonction de nombreux facteurs tels que l'état de forme de l'individu, son âge, son entraînement physique, ou la prise d'un traitement affectant son efficacité musculaire.

L'évolution des caractéristiques d'une foulée au cours du temps peut être représentative de l'évolution de l'état de forme physique d'un individu, en particulier dans le cas d'un individu atteint d'une maladie neuromusculaire ou neuro-dégénérative. Ces maladies, telles que la Dystrophie Musculaire de Duchenne (DMD), se traduisent par une réduction au fur et à mesure de la progression de la maladie des capacités musculaires de l'individu. Cela conduit à une altération des caractéristiques des foulées, qui deviennent typiquement plus lentes, plus courtes, moins nombreuses.

De manière connue, la foulée d'un individu peut être analysée lors de tests, tels que le test de marche de six minutes (6MWT, six minutes walk test), le North Star Ambulation Assessment (test NSAA), ou le test de montée de 4 marches d'escalier chronométré.

Le 6MWT consiste à demander à l'individu de marcher au maximum de ses capacités pendant six minutes, la distance parcourue étant alors analysée. Le 6MWT est utilisé dans de nombreux domaines, tels que l'orthopédie ou diverses pathologies neuro-musculaires, et vise à mesurer l'effort maximal que l'individu est capable de produire, afin de caractériser son état de forme physique par rapport à un groupe normatif. En effet, la puissance maximale développée par l'individu est représentative de son état physique et de la puissance de ses muscles.

Le test NSAA inclue 17 activités fonctionnelles, incluant une marche ou une course de 10 minutes, des changements entre des positions assise, debout et couchée, la montée et la descente de marches, des sauts, etc.

Enfin, le test de montée de 4 marches d'escalier chronométré mesure la durée minimale nécessaire à l'individu pour monter quatre marches d'escalier.

Ces tests peuvent être répétés à différentes échéances, afin de suivre l'évolution de l'état de forme de l'individu. Néanmoins, de tels tests sont contraignants car ils exigent d'être réalisés en milieu contrôlé (généralement dans un centre clinique), sur un parcours particulier (d'une certaine distance, en intérieur, plat, idéalement rectiligne), et en présence d'un personnel qualifié afin de valider les conditions attendues. L'individu doit donc se déplacer régulièrement dans un centre clinique pour réaliser les tests. Par ailleurs, l'individu ne peut réaliser ces tests que lors de sessions ponctuelles, et les tests peuvent être biaisés par des conditions extérieures affectant la performance de l'individu au moment du test (fatigue passagère, motivation et concentration variable d'une session à l'autre, longueur du couloir, adhérence du sol, encouragements donnés pendant le test, etc.). Pendant le 6MWT, l'individu peut, volontairement ou inconsciemment, marcher plus ou moins vite au fil des tests. Le test de montée de 4 marches d'escalier peut être très rapide, de sorte que des biais relatifs au déclenchement de la mesure peuvent être constatés.

Ainsi, les tests évoqués précédemment peuvent ne pas être représentatifs de l'état de forme général de l'individu et la variabilité acceptée pour un individu donné à ces tests, ainsi que la valeur correspondant à un changement cliniquement substantiel, sont très élevés. A titre d'exemple, pour le 6MWT, la variabilité acceptée est d'environ 15% et un changement cliniquement substantiel est de l'ordre de 30 m.

De manière également connue, certaines applications, par exemple de podomètres, de téléphones portables ou de montres connectées, permettent de compter les foulées ou le nombre d'épisodes d'activité d'un individu. La distance parcourue par l'individu peut alors être mesurée par un récepteur GPS embarqué dans de tels dispositifs. Néanmoins, cette mesure manque de précision, et ces applications ne permettent pas de déterminer la trajectoire d'une foulée. Or le nombre de foulées n'est pas toujours représentatif de l'état de santé d'un individu. En effet, ce paramètre est très sensible aux variables extérieures, telles que l'emploi du temps de l'individu, les éventuels changements de son mode de vie, etc. Par ailleurs, un individu peut effectuer un grand nombre de foulées mais à une vitesse très limitée, ou avec une longueur très réduite. Enfin, un affaiblissement ou une amélioration modérée de la puissance musculaire peut ne pas avoir d'impact sur les mouvements les plus courants qui approchent peu la limite de puissance ou d'endurance de l'individu. Un simple comptage du nombre de foulées, de même que la simple indication d'une valeur moyenne de longueur de foulée, ne permet donc pas de caractériser de manière satisfaisante l'état de forme physique d'un individu.

Par ailleurs, le document US 2013/123665 A1 propose d'analyser la trajectoire de foulées d'un individu à partir d'un dispositif attaché sur son pied. Cependant, ce dispositif ne réalise pas une analyse de foulée représentative de la puissance musculaire maximale qu'un individu est capable de développer, et ne permet pas d'analyser l'évolution au cours du temps de l'état de forme physique d'un individu.

Enfin, l'abrégé de SEFERIAN A ET AL: "Longitudinal results of magneto-inertial motion analysis in Duchenne muscular dystrophy ambulant patients",NEUROMUSCULAR DISORDERS, vol. 26, 2016,ISSN: 0960-8966, DOI: 10.1016/J.NMD.2016.06.357, propose une analyse de la foulée de patients atteints de la dystrophie musculaire de Duchenne. Il indique, qu'à partir des mesures d'ActiMyo®, il est possible de reconstruire la trajectoire de l'appareil et de calculer avec précision des variables telles que la vitesse et la longueur du pas. Il propose une évaluation basée sur la fonction de densité de la longueur et de la vitesse du pas, calculée sur des intervalles successifs d'environ 30 jours.

## RESUME DE L'INVENTION

Un but de l'invention est de proposer un équipement d'analyse de foulée d'un piéton en marche qui permette de suivre l'évolution de la forme physique du piéton au cours du temps.

Un autre but de l'invention est de déduire d'une analyse de foulée une information sur la puissance musculaire maximale que l'individu est capable de développer.

Un autre but de l'invention est que cette analyse puisse être réalisée en continu, avec des contraintes moindres pour l'individu.

L'invention est définie par les revendications indépendantes 1 et 12. Des caractéristiques préférentielles de l'équipement revendiqué sont définies dans les revendications dépendantes 2-11.

Selon un premier aspect non revendiqué, il est décrit un procédé d "analyse de foulée d'un piéton en marche, le procédé comprenant des étapes de :

(a) Acquisition, pendant une période d'enregistrement, de mesures de mouvement d'un membre inférieur du piéton,
(b) Détermination, par une unité de traitement de données, de chaque foulée effectuée par le piéton au cours de la période d'enregistrement, en fonction des mesures de mouvement,
(c) Division de la période d'enregistrement en sous-périodes d'enregistrement,
(d) Pour plusieurs foulées détectées, estimation d'une grandeur caractéristique de la foulée, en fonction des mesures de mouvement, dans lequel la grandeur caractéristique est ou dépend d'une longueur de foulée,
(e) Sélection d'au moins une grandeur caractéristique de foulée dans une gamme de percentiles prédéterminée au sein d'un ensemble formé par les grandeurs caractéristiques de foulées estimées, ordonnées par valeurs croissantes et survenues au cours d'une sous-période d'enregistrement,
(f) Répétition de l'étape (e) pour plusieurs sous-périodes d'enregistrement.

Sélectionner, lors de l'étape (e), une grandeur caractéristique de foulée dans une gamme de percentiles prédéterminée, comprise entre le 70$^{ème}$ et le 100$^{ème}$ percentile, pour une sous-période d'enregistrement donnée, permet de disposer d'un indicateur simple et pertinent (en l'occurrence, un percentile de cette grandeur caractéristique) de l'état de forme physique d'un individu au cours d'une sous-période donnée.

Répéter, lors de l'étape (f), l'étape (e) pour plusieurs sous-périodes d'enregistrement, permet de suivre l'évolution de l'état de forme physique d'un individu au cours du temps.

Un percentile élevé de grandeur caractéristique reflète la puissance musculaire maximale que l'individu est capable de développer.

Certaines caractéristiques préférées du procédé sont les suivantes, prises individuellement ou en combinaison :

- La grandeur caractéristique d'une foulée est une vitesse moyenne de la foulée.
- La gamme de percentiles prédéterminée est constituée du 95ème percentile.
- L'acquisition de mesures de mouvement est réalisée par au moins un capteur inertiel, tel qu'un accéléromètre ou un gyromètre.
- Le capteur inertiel est attaché au membre inférieur du piéton, par exemple au niveau d'une cheville du piéton. Une telle disposition présente, notamment par rapport à un capteur attaché à un pied de l'individu, des avantages en termes d'ergonomie, de confort, d'esthétique (capteur dissimulé sous un pantalon) et de sécurité (risque de chute si un capteur attaché au pied s'accroche à un élément extérieur).
- La détermination d'une foulée comprend la détection pour un instant donné d'une accélération de valeur absolue supérieure à un seuil prédéterminé, cet instant définissant le début de la foulée et la fin d'une foulée précédente.
- La longueur d'une foulée est la longueur de la projection d'une trajectoire de la foulée sur un plan horizontal, ou est la longueur curviligne d'une trajectoire de la foulée, ladite trajectoire étant estimée en fonction des mesures de mouvement.

- La vitesse moyenne d'une foulée est un rapport entre la longueur de la foulée et la durée écoulée entre son début et sa fin.
- Le procédé d'analyse de foulée comprend en outre une étape de vérification d'un critère d'appartenance d'une foulée à une séquence continue de plusieurs foulées consécutives, les étapes (d) à (f) du procédé étant effectuées uniquement sur les foulées appartenant à une séquence continue de plusieurs foulées consécutives. Une telle étape permet d'analyser uniquement les foulées représentatives d'une marche naturelle et continue de l'individu, sans que l'analyse soit biaisée par des piétinements, des foulées isolées ou des petits déplacements.
- Une foulée appartient à une séquence continue de plusieurs foulées consécutives lorsque la durée entre le début de ladite foulée et le début d'une foulée précédente ou suivante est comprise entre 0 et 10 secondes, de préférence inférieure à 3 secondes, et que ladite foulée fait partie d'une séquence d'au moins deux foulées consécutives, de préférence d'au moins six foulées consécutives.
- Une sous-période d'enregistrement présente une durée supérieure à 2 jours, de préférence supérieure à 15 jours, et/ou une durée cumulée d'enregistrement supérieure à 5 heures, 10 heures ou 50 heures, de préférence supérieure à 180 heures. Ces critères permettent de définir une sous-période comportant une quantité de données suffisante pour être représentatives de l'activité quotidienne de l'individu, sans que l'état de forme de ce dernier soit susceptible d'être modifié de façon conséquente pendant cette durée.

Selon un deuxième aspect, revendiqué par la revendication indépendante 1, l'invention concerne un équipement d'analyse de foulée d'un piéton en marche, l'équipement comprenant :

- Au moins un capteur inertiel pour acquérir, pendant une période d'enregistrement, des mesures de mouvement d'un membre inférieur du piéton,
- Une unité de traitement de données configurée pour :

  o Déterminer chaque foulée effectuée par le piéton au cours de la période d'enregistrement, en fonction des mesures de mouvement,
  o Diviser la période d'enregistrement en sous-périodes,
  o Pour plusieurs foulées détectées, estimer une grandeur caractéristique de la foulée, en fonction des mesures de mouvement, la grandeur caractéristique étant ou dépendant d'une longueur de foulée,
  o Sélectionner au moins une grandeur caractéristique de foulée dans une gamme de percentiles prédéterminée comprise entre le 70ème et le 100ème percentile dans un ensemble formé par les grandeurs caractéristiques de foulée estimées, ordonnées par valeurs croissantes et survenues au cours d'une des sous-périodes,
  o Répéter l'étape précédente pour plusieurs sous-périodes.

Selon un troisième aspect, revendiqué par la revendication indépendante 12, l'invention concerne un produit programme d'ordinateur comprenant des instructions de code pour l'exécution d'un procédé d'analyse de foulée d'un piéton en marche selon le premier aspect lorsque ce programme est exécuté par un processeur.

## PRÉSENTATION DES FIGURES

D'autres aspects, buts et avantages de la présente invention apparaîtront à la lecture de la description détaillée qui va suivre, donnée à titre d'exemple non limitatif, qui sera illustrée par les figures suivantes :

- La figure 1, déjà commentée, est un schéma représentant des foulées d'un individu.
- La figure 2 est un schéma représentant un équipement d'analyse de foulée d'un piéton en marche conforme à l'nvention.
- La figure 3 est un schéma représentant un équipement d'analyse de foulée d'un piéton en marche conforme à l'nvention, attaché à un membre inférieur d'un piéton.
- La figure 4 est un graphique représentant un exemple de trajectoire d'une cheville pendant plusieurs foulées.

## DESCRIPTION DETAILLEE D'UN MODE DE RÉALISATION

Equipement d'analyse de foulée

En référence à la figure 2, un équipement d'analyse de foulée 10 comprend des capteurs inertiels 11. Les capteurs inertiels 11 peuvent comprendre un accéléromètre et un gyromètre. De préférence, les capteurs inertiels 11 comprennent une centrale à inertie comprenant au moins trois accéléromètres et trois gyromètres, par exemple de type MEMS. L'équipement d'analyse de foulée 10 peut également comprendre une batterie, un magnétomètre 14, un altimètre et/ou un GPS.

L'équipement d'analyse de foulée 10 peut comprendre une unité de traitement de données 12 (typiquement un processeur) pour la mise en œuvre en temps réel des traitements de données. L'équipement d'analyse de foulée 10 peut également comprendre des moyens de stockage 13 (par exemple une mémoire de type flash) capable de stocker les mesures effectuées et/ou des données à traiter ou traitées par l'unité de traitement de données 12. L'équipement d'analyse de foulée 10 peut également inclure des moyens de communication 15 pour transmettre une position estimée. Par exemple, la position du porteur peut être envoyée à un terminal mobile pour être affichée dans une interface d'un logiciel de navigation.

En variante, l'équipement d'analyse de foulée 10 peut comprendre des moyens de communication 15 permettant de transmettre les mesures à un dispositif externe tel qu'un terminal mobile, ou un serveur distant. Ces moyens de communication 15 peuvent mettre en œuvre une communication sans fil à courte portée par exemple Bluetooth ou Wifi (en particulier dans un mode de réalisation avec un terminal mobile), voire être des moyens de connexion à un réseau mobile (typiquement UMTS/LTE) pour une communication à longue distance. Il est à noter que les moyens de communication 15 peuvent être par exemple une connectique filaire (typiquement USB) pour transférer les données des moyens de stockage 13 de données locales à ceux d'un terminal mobile ou d'un serveur. Le terminal mobile ou serveur distant comprend alors une unité de traitement de données 12 (typiquement un processeur) pour la mise en œuvre des traitements de données.

Dans la suite de la présente description, on verra que l'unité de traitement de données 12 respectivement de l'équipement d'analyse de foulée 10, d'un terminal mobile et d'un serveur distant, peut indifféremment et selon les applications réaliser tout ou partie des étapes du procédé.

L'équipement d'analyse de foulée 10 peut en outre comprendre un boîtier 17 comprenant les capteurs inertiels 11. Le boîtier 17 peut en outre comprendre l'éventuelle unité de traitement de données 12, moyens de stockage 13 et moyens de communication 15.

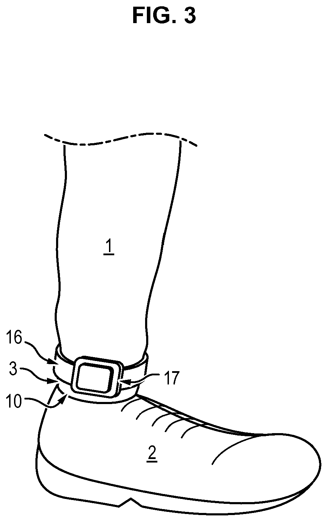

En référence à la figure 3, le piéton présente au moins un membre inférieur 1, tel qu'une jambe. On comprendra que l'un ou l'autre ou chacun des deux membres inférieurs 1 du piéton peut être équipé d'un équipement d'analyse de foulée 10.

L'équipement d'analyse de foulée 10 peut en outre comprendre des moyens d'attache 16 au membre inférieur 1, par exemple au pied, à la cheville, au tibia, ou encore à la cuisse, du piéton. Par exemple, l'équipement d'analyse de foulée 10 comprend des moyens d'attache 16 au niveau d'une cheville 3 du piéton. Les moyens d'attache 16 du boîtier 17 peuvent comprendre une sangle passée autour du membre inférieur 1, ou une bande auto agrippante qui enserre le membre inférieur 1 et permet sa liaison solidaire avec le boîtier 17 (donc avec les capteurs inertiels 11 qu'il comporte). Ainsi, les capteurs inertiels 11 présentent un mouvement sensiblement identique dans le référentiel terrestre au mouvement de la cheville 3 du piéton. Par ailleurs, le boîtier 17 et ses moyens d'attache 16 peuvent rester dissimulés et protégés sous un pantalon de l'individu. Lindividu peut porter le boîtier 17 au quotidien et de manière continue, ce qui augmente la pertinence de l'analyse de foulées. Néanmoins, ces capteurs inertiels 11 peuvent également être disposés par exemple au niveau du tibia du piéton, ou de sa cuisse.

Procédé d'analyse de foulée

Un procédé d'analyse de foulée d'un piéton en marche comprend les étapes suivantes.

Le capteur inertiel 11 est de préférence attaché au membre inférieur 1 du piéton, par exemple à son pied, à sa cheville, à son tibia, ou encore à sa cuisse. De préférence, le capteur inertiel 11 est attaché au niveau d'une cheville 3 du piéton.

Le procédé d'analyse de foulée comprend une première étape (a) d'acquisition, pendant une période d'enregistrement, de mesures de mouvement d'un membre inférieur 1 du piéton. L'acquisition est de préférence réalisée par au moins un capteur inertiel 11, tel qu'un accéléromètre ou un gyromètre. Les mesures de mouvement sont par exemple des mesures d'accélération et de vitesse angulaire de la cheville 3 du piéton.

La période d'enregistrement correspond à la durée pendant laquelle l'individu est susceptible de porter l'équipement d'analyse de foulée 10, les capteurs inertiels 11 pouvant alors réaliser des mesures inertielles. Par exemple, un individu peut porter le boîtier 17 en journée, soit environ douze heures par jour. Dans ce cas, pour une période d'enregistrement de 15 jours, l'équipement d'analyse de foulée 10 aura réalisé 180 heures d'enregistrement. Il est également possible que l'individu porte l'équipement d'analyse de foulée 10 douze heures par jours mais seulement certains jours, par exemple un jour sur deux. Dans ce cas, pour une même période d'enregistrement de 15 jours, l'équipement d'analyse de foulée 10 aura réalisé seulement 90 heures d'enregistrement.

Le procédé d'analyse de foulée comprend une deuxième étape (b) de détermination, par l'unité de traitement de données 12, de chaque foulée effectuée par le piéton au cours de la période d'enregistrement, en fonction des mesures de mouvement. Cela consiste à déterminer qu'une foulée a été effectuée, ainsi qu'à déterminer l'instant de début de foulée et l'instant de fin de foulée.

Dans le cas d'un équipement d'analyse de foulée attaché à une chaussure du piéton, la détermination de la foulée peut être effectuée en recherchant les phases de vitesse angulaire nulle et de norme d'accélération égale à la gravité. Cette méthode est connue notamment dans le cadre de méthodes ZUPT (Zero velocity update) par exemple [Foxlin, Eric, "Pedestrian Tracking with Shoe-Mounted Inertial Sensors", IEEE Computer Society, Nov./Dec. 2005, pp. 38-46].

Dans le cas d'un équipement d'analyse de foulée 10 situé sur la cheville 3, les accélération et vitesse angulaire mesurées ne sont jamais nulles, y compris lorsque le pied 2 est posé au sol, car il y a une rotation autour du talon pendant la phase où le pied 2 est en contact avec le sol. Par conséquent, les méthodes telles que la méthode ZUPT ne peuvent être appliquées directement et il est préférable de s'appuyer sur d'autres critères que ceux de mesures nulles ou quasi-nulles afin de déterminer une foulée.

Selon un mode de réalisation préféré, la détermination d'une foulée comprend la détection pour un instant donné d'une accélération de valeur absolue supérieure à un seuil prédéterminé, cet instant définissant le début de la foulée et la fin de la foulée précédente. En effet, au moment de l'impact du pied 2 sur le sol, un choc se produit sur tout le membre inférieur 1. La valeur absolue de l'accélération mesurée par l'accéléromètre est alors susceptible de dépasser le seuil prédéterminé. Ce seuil peut être par exemple compris entre 5 m/s$^2$ et 50 m/s$^2$, est être par exemple égal à 15 m/s$^2$.

Afin d'améliorer la précision de la détection d'une foulée, il est possible de combiner la détection d'une accélération de valeur absolue supérieure à un seuil prédéterminé avec un deuxième critère de détermination de foulée. Ce deuxième critère peut dépendre de la durée écoulée depuis le choc, de l'accélération spécifique mesurée pendant une certaine durée, du pic d'accélération correspondant au choc, de la vitesse angulaire mesurée pendant une durée écoulée depuis le choc, et/ou de la variation de la vitesse angulaire pendant une durée écoulée depuis le choc.

Plus précisément, ce deuxième critère peut se présenter sous la forme d'une minimisation d'une expression dépendant notamment d'un ou de plusieurs des paramètres ci-dessus. Le minimum de l'expression peut être recherché dans une certaine durée après le choc, par exemple une durée comprise entre 0 secondes et 1 seconde après le choc, de préférence une durée de 0,5 secondes après le choc. L'expression à minimiser peut s'écrire sous la forme :

$$\Delta t_{choc} + (|accel|_{X\,ms} - Choc) + \alpha * |Vang|_{X\,ms} + \beta * \Delta Vang_{X\,ms},$$

où

*Choc* représente la valeur du dernier pic d'accélération spécifique détecté, c'est-à-dire ayant une norme supérieure à seuil prédéterminé de 1g, de préférence supérieure à 1,05g,

$\Delta t_{choc}$ représente la durée écoulée entre le choc détecté et l'instant courant par l'accélération de valeur absolue supérieure au seuil prédéterminé,

|*accel*| représente la valeur absolue moyenne de l'accélération spécifique mesurée en m/s$^2$ pendant X ms, X étant de préférence compris entre 10 et 500, de préférence égal à 40,

$\alpha$ est un nombre, par exemple compris entre 1 et 100, de préférence égal à 12,

|*Vang*|$_{X\,ms}$ représente la moyenne de la valeur absolue de la vitesse angulaire mesuré en °/s sur X ms, X étant de préférence compris entre 10 et 500, de préférence égal à 40,

$\beta$ est un nombre, par exemple compris entre 1 et 100, de préférence égal à 10,

$\Delta Vang_{X\,ms}$ représente la variation de la vitesse angulaire dans l'axe perpendiculaire à la jambe et au pied et mesurée en °/s pendant X ms, X étant de préférence compris entre 10 et 500, de préférence égal à 40.

Le début d'une foulée peut correspondre à l'instant où l'expression ci-dessous est minimisée, cet instant correspondant également à la fin de la foulée précédente. La combinaison de la détection d'une accélération de valeur absolue supérieure à un seuil prédéterminé et de la minimisation de l'expression ci-dessus permet de déterminer une foulée de manière précise et consistante.

En variante, des méthodes d'apprentissage automatique tels qu'un réseau de neurones, une forêt aléatoire, une régression ou tout autre algorithme d'apprentissage statistique connu, peuvent être utilisées pour identifier un pattern typique de variations de mesures, ce pattern permettant de déterminer une foulée.

Le procédé d'analyse de foulée comprend une troisième étape (c) de division de la période d'enregistrement en sous-périodes d'enregistrement. Cette étape permet de suivre l'évolution des variables mesurées, donc de l'état de forme d'un individu, sous-période après sous-période. Par exemple, dans le cas où la période d'enregistrement est de six mois, il peut être intéressant de disposer de l'évolution de l'état de forme de l'individu mois après mois, la période d'enregistrement étant alors divisée en six sous-périodes d'un mois, plutôt que de disposer d'un seul résultat pour l'ensemble de la période d'enregistrement.

Une sous-période d'enregistrement doit présenter une durée et une durée cumulée d'enregistrement suffisamment longues pour que l'analyse des foulées qu'elle comporte soit représentative de l'état de forme d'un individu. Selon un mode préféré de réalisation, une sous-période d'enregistrement présente une durée supérieure à 2 jours, de préférence supérieure à 15 jours, et/ou une durée cumulée d'enregistrement supérieure à 5 heures, 10 heures ou 50 heures, de préférence supérieure à 180 heures.

En effet, si un individu n'a pas porté le dispositif d'analyse de foulée 10 pendant 15 jours, la sous-période d'enregistrement correspondante aurait une durée de 15 jours, mais une durée cumulée d'enregistrement de 0 heures. Une telle sous-période ne comprendrait aucune foulée, et ne serait donc pas représentative de l'état de forme d'un individu.

Une durée de 15 jours et de 180 heures d'enregistrement est a priori suffisamment longue pour garantir une quantité de données suffisante pour être représentatives de l'activité quotidienne de l'individu, tout en étant suffisamment courte pour que l'état de forme de l'individu ne soit pas sensiblement modifié pendant cette durée. Une telle durée permet également de diminuer la variabilité des mesures, tout en ne représentant pas une contrainte trop importante pour l'individu portant l'équipement d'analyse de foulée 10.

En variante, seul l'un ou l'autre de ces critères de durée et de durée cumulée d'enregistrement de la sous-période doit être respecté.

Le procédé d'analyse de foulée comprend une quatrième étape (d) qui consiste, pour plusieurs foulées détectées, à estimer une grandeur caractéristique de la foulée, en fonction des mesures de mouvement. La grandeur caractéristique estimée peut par exemple être une vitesse moyenne de foulée, ou encore une longueur de foulée.

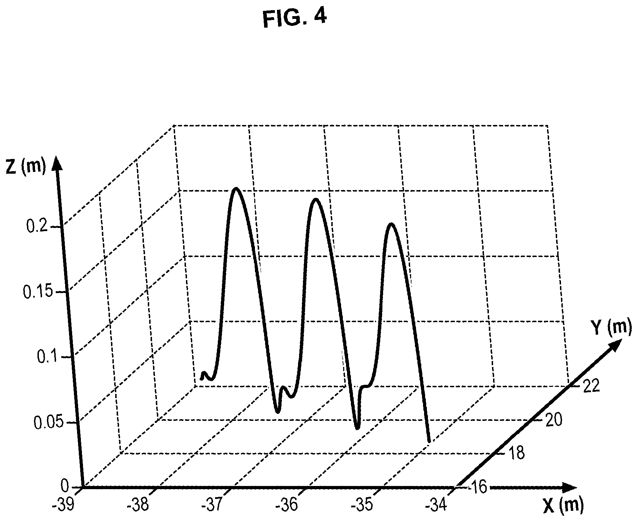

De préférence, l'unité de traitement de données 12 réalise une estimation d'une trajectoire de la cheville 3 au cours de la période d'enregistrement. Cette estimation peut être effectuée à partir d'une double intégration des mesures de mouvement, la double intégration étant de préférence recalée à chaque fois que le pied 2 touche le sol. Un dispositif permettant de réaliser une estimation d'une trajectoire de la cheville est décrit dans le document FR 3 042 266. La figure 4 représente un exemple de représentation de trajectoire estimée sur trois foulées consécutives.

Selon un mode de réalisation préféré, la longueur d'une foulée est la longueur de la projection de la trajectoire de la foulée sur un plan horizontal (c'est-à-dire un plan parallèle à un sol sur lequel l'individu portant l'équipement d'analyse de foulée 10 marche), la trajectoire étant estimée en fonction des mesures de mouvement, comme décrit ci-dessus. En variante, la longueur d'une foulée peut être par exemple la longueur curviligne de la trajectoire estimée, sans projeter celle-ci dans le plan horizontal.

La vitesse moyenne d'une foulée peut être définie par le rapport entre la longueur de la foulée et sa durée, la durée d'une foulée correspondant à la durée écoulée entre son début et sa fin.

La détermination d'une vitesse moyenne de foulée présente plusieurs avantages par rapport au cas où la vitesse instantanée de la foulée serait considérée en continu pendant la marche, ou échantillonnée à haute fréquence. En effet, les foulées très lentes, qui ont une durée plus longue que les foulées rapides, seraient alors associées à un nombre supérieur de vitesses par rapport aux foulées plus rapides, ce qui altérerait l'analyse des résultats. Par ailleurs, la dérivation de la trajectoire permettant d'estimer une vitesse instantanée engendrerait un bruit susceptible d'effecter les valeurs des vitesses instantanées estimées. Enfin, affecter une vitesse moyenne unique à une foulée donnée permet de simplifier le traitement des données en réduisant le nombre de données à traiter, dans le but de réaliser un calcul statistique ultérieur sur ces données.

Un autre mode de réalisation consiste à considérer la vitesse tout au long de chaque trajectoire à la fréquence d'échantillonnage permise par le calcul, dans un mode de réalisation préféré entre 50Hz et 1000hz, de préférence au voisinage de 100Hz.

Le procédé d'analyse de foulée comprend une cinquième étape (e) de sélection d'au moins une grandeur caractéristique (par exemple une vitesse moyenne et/ou longueur) de foulée dans une gamme de percentiles prédéterminée au sein d'un ensemble formé par les grandeurs caractéristiques de foulées estimées, ordonnées par valeurs croissantes et survenues au cours d'une sous-période d'enregistrement.

Une foulée peut être associée à sa date, son heure, et/ou son instant de début et/ou de fin, ce qui permet de l'associer à une sous-période d'enregistrement donnée. Une foulée peut en outre être associée à sa grandeur caractéristique (par exemple sa vitesse moyenne et/ou sa longueur). Les foulées d'une sous-période donnée peuvent être ordonnées par valeurs croissantes de grandeurs caractéristiques.

Un calcul statistique peut alors être réalisé sur un ensemble formé par les grandeurs caractéristiques (par exemple les vitesses moyennes et/ou longueurs) de foulées estimées, ordonnées par valeurs croissantes et survenues au cours d'une sous-période d'enregistrement. Notamment, il est possible de calculer un percentile de cette grandeur caractéristique, par rapport à l'ensemble des grandeurs caractéristiques des foulées de la sous-période d'enregistrement. Par exemple, il est possible de calculer le 50$^{\text{ème}}$ percentile, qui correspond à la médiane, le 80$^{\text{ème}}$ percentile, le 95ème percentile, ou toute autre valeur de percentile de la grandeur caractéristique de foulée pour une sous-période donnée.

Une gamme de percentiles prédéterminée peut être définie, cette gamme de percentiles présentant un intérêt pour l'analyse de foulée. En particulier, les percentiles élevés sont représentatifs de l'effort maximal et de la puissance musculaire maximale qu'un individu est capable de développer, car ils reflètent les foulées les plus rapides et/ou les plus longues d'un individu, la vitesse de marche étant contrainte par l'état de force musculaire. Ces percentiles élevés sont donc particulièrement sensibles à l'état de forme d'un individu. Le terme de percentile élevé est utilisé dans la demande pour désigner un percentile supérieur au 70ème percentile.

Un tel procédé d'analyse de foulée à partir d'une gamme de percentiles élevés est particulièrement adapté à la marche, car un individu réalise statistiquement un certain nombre de foulées plus longues et/ou plus rapides dans la vie de tous les jours. En particulier, les quelques pourcents de foulées les plus longues et/ou les plus rapides sont représentatives de la puissance maximale que l'individu est capable de développer. A titre d'exemple, des études ont révélé une corrélation entre les mesures de test d'efforts tels que le 6MWT, et le 95ème percentile de longueur et/ou de vitesse de foulée chez un même individu.

La gamme de percentiles prédéterminée est comprise entre le 70ème et le 100ème percentile. La gamme de percentiles prédéterminée peut être constituée du 95ème percentile.

Le procédé d'analyse de foulée comprend une cinquième étape (f) de répétition de l'étape (e) pour plusieurs sous-périodes d'enregistrement.

Une telle étape (f) permet d'accéder aux grandeurs caractéristiques de foulée dans la gamme de percentiles prédéterminée pour plusieurs sous-périodes d'enregistrement. La gamme de percentiles prédéterminée est la même pour chaque sous-période pour laquelle l'étape (e) est répétée. Ainsi, il est possible de suivre l'évolution de la grandeur caractéristique de foulée, sous-période après sous-période. L'évolution de l'état de forme au cours du temps de l'individu portant l'équipement d'analyse de foulée 10 peut alors être suivie.

Dans un mode de réalisation préféré, le procédé d'analyse de foulée comprend en outre une étape de vérification d'un critère d'appartenance d'une foulée à une séquence continue de plusieurs foulées consécutives, les étapes (d) à (f) du procédé étant effectuées uniquement sur les foulées appartenant à une séquence continue de plusieurs foulées consécutives.

Une telle étape permet d'analyser uniquement les données provenant d'une marche continue et naturelle, lorsque l'individu est sur sa lancée. Ainsi, les données ne risquent pas d'être biaisées par une quantité importante de piétinements, de foulées isolées ou de micro-déplacements de l'individu, ces foulées n'étant pas représentatives de l'état de forme de l'individu. Ainsi, les foulées considérées pour le calcul de la gamme de percentile prédéterminée, en particulier du 95ème percentile, ne prennent pas en compte les foulées isolées ou piétinements, ce qui élimine le biais de mesure correspondant.

De préférence, une foulée appartient à une séquence continue de plusieurs foulées consécutives lorsque la durée entre le début de ladite foulée et le début d'une foulée précédente ou suivante est comprise entre 0 et 10 secondes, de préférence inférieure à 3 secondes, et que ladite foulée fait partie d'une séquence d'au moins deux foulées consécutives, de préférence d'au moins six foulées consécutives.

En effet, une durée entre deux foulées supérieure par exemple à 0,5 secondes, 3 secondes ou 10 secondes, traduit un arrêt probable de l'individu entre deux foulées. Les foulées correspondent alors à des piétinements, n'appartiennent pas à une même séquence de marche et ne reflètent pas l'état de forme de l'individu.

Par ailleurs, lors d'une séquence de moins de par exemple dix, six, ou deux foulées consécutives, l'individu n'est a priori pas dans un rythme normal de marche, mais dans un rythme lent, correspondant à un piétinement ou à un petit déplacement.

La combinaison de ces deux critères permet de ne pas traiter les foulées qui ne seraient pas caractéristiques d'une marche continue de l'individu et de la puissance musculaire qu'il est capable de développer. En variante, un seul des deux critères ci-dessus doit être vérifié pour que les étapes (d) à (f) du procédé soient effectuées.

Dans le même objectif de ne traiter que les foulées représentatives de l'état de forme de l'iindividu, il est possible d'identifier des foulées comportant une erreur, par exemple dans le calcul de la trajectoire ou dans la détermination de leur début et de leur fin. A cet effet, la trajectoire d'une foulée peut être comparée à une ou plusieurs trajectoires de référence. La forme de la trajectoire peut être étudiée, l'existence d'un maximum de position verticale autour du milieu de la foulée peut être vérifiée, des ratios entre hauteur et longueur de trajectoire peuvent être définis. Les foulées s'éloignant de manière excessive des caractéristiques attendues au vu des trajectoires de référence sont alors identifiées comme erronées, les étapes (d) à (f) du procédé n'étant pas effectuées sur ces foulées. Ainsi, l'analyse de données n'est pas biaisée par d'éventuelles erreurs de calcul de trajectoire, de détermination de foulée, ou autre.

En variante, un algorithme d âpprentissage automatique tel qu'un réseau de neurones, une forêt aléatoire, un SVM ou toute autre méthode connue, peut être utilisé pour comparer une foulée à une base de données de foulées de référence, et identifier ainsi une foulée erronée.

En outre, il est possible d'identifier la première foulée et la dernière foulée de chaque marche, les étapes (d) à (f) du procédé n'étant pas effectuées sur ces foulées. En effet, ces foulées ne sont pas non plus représentatives d'une marche continue d'un individu.

Il est également possible d'identifier les foulées comportant un changement de cap, les étapes (d) à (f) du procédé n'étant pas effectuées sur ces foulées. Ainsi, seules les foulées appartenant à une marche en ligne droite sont conservées. En effet, un changement de cap entraîne un ralentissement, et une foulée comportant un changement de cap n'est donc pas représentative d'une marche continue et lancée d'un individu. De plus, une telle foulée est susceptible d'autre moins bien déterminée, le pattern de rotation et d'accélération de la foulée étant altéré par le changement de cap.

L'étape (a) du procédé d'analyse de foulée peut être mise en œuvre par au moins un capteur inertiel 11 de l'équipement d'analyse de foulée 10.

Les étapes (b) à (f) du procédé d'analyse de foulée, ainsi que les potentielles étapes supplémentaires décrites ci-dessus, peuvent être mises en œuvre par l'unité de traitement de données 12 de l'équipement d'analyse de foulée 10.

**Revendications**

1. Equipement d'analyse de foulée (10) d'un piéton en marche, l'équipement (10) comprenant :

   - Au moins un capteur inertiel (11) pour acquérir, pendant une période d'enregistrement, des mesures de mouvement d'un membre inférieur (1) du piéton,
   - Une unité de traitement de données (12) configurée pour :

     o Déterminer chaque foulée effectuée par le piéton au cours de la période d'enregistrement, en fonction des mesures de mouvement,
     o Diviser la période d'enregistrement en sous-périodes,
     o Pour plusieurs foulées détectées, estimer une grandeur caractéristique de la foulée, en fonction des mesures de mouvement, la grandeur caractéristique étant ou dépendant d'une longueur de foulée,
     o Sélectionner au moins une grandeur caractéristique de foulée dans une gamme de percentiles prédéterminée comprise entre le 70$^{ème}$ et le 100$^{ème}$ percentile dans un ensemble formé par les grandeurs caractéristiques de foulée estimées, ordonnées par valeurs croissantes et survenues au cours d'une des sous-périodes,
     o Répéter l'étape précédente pour plusieurs sous-périodes.

2. Equipement (10) selon la revendication précédente, dans lequel la grandeur caractéristique d'une foulée est une vitesse moyenne de la foulée.

3. Equipement (10) selon l'une des revendications précédentes, dans lequel la gamme de percentiles prédéterminée est constituée du 95ème percentile.

4. Equipement (10) selon l'une des revendications précédentes, dans lequel le capteur inertiel (11) comprend un accéléromètre et/ou un gyromètre.

5. Equipement (10) selon l'une des revendications précédentes, dans lequel le capteur inertiel (11) est configuré pour être attaché au membre inférieur (1) du piéton, par exemple au niveau d'une cheville (3) du piéton.

6. Equipement (10) selon l'une des revendications précédentes, dans lequel la détermination d'une foulée comprend la détection pour un instant donné d'une accélération de valeur absolue supérieure à un seuil prédéterminé, cet instant définissant le début de la foulée et la fin d'une foulée précédente.

7. Equipement (10) selon l'une des revendications précédentes, dans lequel la longueur d'une foulée est la longueur de la projection d'une trajectoire de la foulée sur un plan horizontal, ou est la longueur curviligne d'une trajectoire de la foulée, ladite trajectoire étant estimée en fonction des mesures de mouvement.

8. Equipement (10) selon la revendication 6 dans sa dépendance à la revendication 2, dans lequel la vitesse moyenne d'une foulée est un rapport entre la longueur de la foulée et la durée écoulée entre son début et sa fin.

9. Equipement (10) selon l'une des revendications précédentes, dans lequel l'unité de traitement de données (12) est en outre configurée pour vérifier un critère d'appartenance d'une foulée à une séquence continue de plusieurs foulées consécutives, et pour ne mettre en œuvre les étapes d'estimation, de sélection et de répétition que sur les foulées appartenant à une séquence continue de plusieurs foulées consécutives.

10. Equipement (10) selon la revendication 9, dans lequel une foulée appartient à une séquence continue de plusieurs foulées consécutives lorsque la durée entre le début de ladite foulée et le début d'une foulée précédente ou suivante est comprise entre 0 et 10 secondes, de préférence inférieure à 3 secondes, et que ladite foulée fait partie d'une séquence d'au moins deux foulées consécutives, de préférence d'au moins six foulées consécutives.

**11.** Equipement (10) selon l'une des revendications précédentes, dans lequel une sous-période d'enregistrement présente une durée supérieure à 2 jours, de préférence supérieure à 15 jours, et/ou une durée cumulée d'enregistrement supérieure à 5 heures, 10 heures ou 50 heures, de préférence supérieure à 180 heures.

**12.** Produit programme d'ordinateur comprenant des instructions de code pour l'exécution, lorsque ledit programme est exécuté sur un ordinateur, d'un procédé d'analyse de foulée d'un piéton en marche comprenant des étapes de :

(a) Réception de mesures de mouvement d'un membre inférieur (1) du piéton acquises pendant une période d'enregistrement,

(b) Détermination, par une unité de traitement de données (12), de chaque foulée effectuée par le piéton au cours de la période d'enregistrement, en fonction des mesures de mouvement,

(c) Division de la période d'enregistrement en sous-périodes d'enregistrement,

(d) Pour plusieurs foulées détectées, estimation d'une grandeur caractéristique de la foulée, en fonction des mesures de mouvement, dans lequel la grandeur caractéristique est ou dépend d'une longueur de la foulée,

(e) Sélection d'au moins une grandeur caractéristique de foulée dans une gamme de percentiles prédéterminée au sein d'un ensemble formé par les grandeurs caractéristiques estimées, ordonnées par valeurs croissantes et survenues au cours d'une sous-période d'enregistrement, la gamme de percentiles prédéterminée étant comprise entre le 70$^{ème}$ et le 100$^{ème}$ percentile,

(f) Répétition de l'étape (e) pour plusieurs sous-périodes d'enregistrement.

**Patentansprüche**

**1.** Ausrüstung (10) zur Schrittanalyse eines gehenden Fußgängers, wobei die Ausrüstung (10) umfasst:

- mindestens einen Trägheitssensor (11), um während eines Aufzeichnungszeitraums Bewegungsmessungen einer unteren Gliedmaße (1) des Fußgängers zu erfassen,
- eine Datenverarbeitungseinheit (12), die ausgelegt ist, um:

  ○ jeden vom Fußgänger während des Aufzeichnungszeitraums ausgeführten Schritt in Abhängigkeit von den Bewegungsmessungen zu bestimmen,
  ○ jeden Aufzeichnungszeitraum in Unterzeiträume zu unterteilen,
  ○ für mehrere erfasste Schritte eine charakteristische Größe des Schrittes in Abhängigkeit von den Bewegungsmessungen zu schätzen, wobei die charakteristische Größe eine Schrittlänge ist oder davon abhängt,
  ○ mindestens eine charakteristische Schrittgröße aus einem vorbestimmten Perzentilbereich auszuwählen, der zwischen dem 70. und 100. Perzentil liegt, aus einer Gruppe, die von geschätzten charakteristischen Schrittgrößen gebildet wird, die nach steigenden Werten geordnet sind und während einem der Unterzeiträume aufgetreten sind,
  ○ den vorangehenden Schritt für mehrere Unterzeiträume zu wiederholen.

**2.** Ausrüstung (10) nach vorstehendem Anspruch, wobei die charakteristische Größe eines Schrittes eine durchschnittliche Geschwindigkeit des Schrittes ist.

**3.** Ausrüstung (10) nach einem der vorstehenden Ansprüche, wobei der vorbestimmte Perzentilbereich aus dem 95. Perzentil besteht.

**4.** Ausrüstung (10) nach einem der vorstehenden Ansprüche, wobei der Trägheitssensor (11) einen Beschleunigungsmesser und/oder ein Gyrometer umfasst.

**5.** Ausrüstung (10) nach einem der vorstehenden Ansprüche, wobei der Trägheitssensor (11) ausgelegt ist, um an der unteren Gliedmaße (1) des Fußgängers, beispielsweise an einem Knöchel (3) des Fußgängers, angebracht zu sein.

**6.** Ausrüstung (10) nach einem der vorstehenden Ansprüche, wobei die Bestimmung eines Schrittes die Erfassung einer Beschleunigung mit einem Absolutwert, der größer als ein vorbestimmter Schwellenwert ist, für einen bestimmten Zeitpunkt umfasst, wobei dieser Zeitpunkt den Beginn des Schrittes und das Ende eines vorherigen Schrittes definiert.

**7.** Ausrüstung (10) nach einem der vorstehenden Ansprüche, wobei die Länge eines Schrittes die Länge der Projektion einer Bahn des Schrittes auf eine horizontale Ebene ist oder die gekrümmte Länge einer Bahn eines Schrittes ist, wobei die Bahn in Abhängigkeit von den Bewegungsmessungen geschätzt wird.

8. Ausrüstung (10) nach Anspruch 6 in Abhängigkeit von Anspruch 2, wobei die Durchschnittsgeschwindigkeit eines Schrittes ein Verhältnis zwischen der Länge des Schrittes und der zwischen seinem Beginn und seinem Ende verstrichenen Zeit ist.

9. Ausrüstung (10) nach einem der vorstehenden Ansprüche, wobei die Datenverarbeitungseinheit (12) ferner ausgelegt ist, um ein Kriterium der Zugehörigkeit eines Schrittes zu einer fortlaufenden Sequenz mehrerer aufeinanderfolgender Schritte zu überprüfen, und die Schritte des Schätzens, des Auswählens und des Wiederholens nur für Schritte durchzuführen, die zu einer fortlaufenden Sequenz mehrerer aufeinanderfolgender Schritte gehören.

10. Ausrüstung (10) nach Anspruch 9, wobei ein Schritt zu einer fortlaufenden Sequenz mehrerer aufeinanderfolgender Schritte gehört, wenn die Dauer zwischen dem Beginn des Schrittes und dem Beginn eines vorhergehenden oder nachfolgenden Schrittes zwischen 0 und 10 Sekunden, vorzugsweise bei weniger als 3 Sekunden, liegt und wenn der Schritt Teil einer Sequenz von mindestens zwei aufeinanderfolgenden Schritten, vorzugsweise von mindestens sechs aufeinanderfolgenden Schritten, ist.

11. Ausrüstung (10) nach einem der vorstehenden Ansprüche, wobei ein Aufzeichnungsunterzeitraum eine Dauer von mehr als 2 Tagen, vorzugsweise von mehr als 15 Tagen, und/oder eine kumulierte Aufzeichnungsdauer von mehr als 5 Stunden, 10 Stunden oder 50 Stunden, vorzugsweise von mehr als 180 Stunden, aufweist.

12. Computerprogrammprodukt, das Codebefehle zur Durchführung eines Verfahrens zur Schrittanalyse eines gehenden Fußgängers umfasst, wenn das Programm auf einem Computer ausgeführt wird, umfassend folgende Schritte:

(a) Empfangen von Bewegungsmessungen einer unteren Gliedmaße (1) des Fußgängers während eines Aufzeichnungszeitraums,
(b) Bestimmen, durch eine Datenverarbeitungseinheit (12), jedes vom Fußgänger während des Aufzeichnungszeitraums ausgeführten Schrittes in Abhängigkeit von den Bewegungsmessungen,
(c) Unterteilen des Aufzeichnungszeitraums in Aufzeichnungsunterzeiträume,
(d) Schätzen, für mehrere erfasste Schritte, einer charakteristischen Größe des Schrittes in Abhängigkeit von den Bewegungsmessungen, wobei die charakteristische Größe eine Schrittlänge ist oder davon abhängt,
(e) Auswählen mindestens einer charakteristischen Schrittgröße aus einem vorbestimmten Perzentilbereich aus einer Menge, die von den geschätzten charakteristischen Größen gebildet wird, geordnet nach steigenden Werten und aufgetreten während eines Aufzeichnungsunterzeitraums, wobei der vorbestimmte Perzentilbereich zwischen dem 70. und dem 100. Perzentil liegt,
(f) Wiederholen von Schritt (e) für mehrere Aufzeichnungsunterzeiträume.

**Claims**

1. Equipment (10) for analyzing the stride of a walking pedestrian, the equipment (10) comprising:

- At least one inertial sensor (11) to acquire, during a recording period, measurements of a motion of a lower limb (1) of the pedestrian,
- A data processing unit (12) configured to:

o Determine each stride made by the pedestrian during the recording period, based on the motion measurements,
o Divide the recording period into sub-periods,
o For several detected strides, estimate a characteristic quantity of the stride, based on the motion measurements, the characteristic quantity being or depending on a stride length,
o Select at least one characteristic stride quantity in a predetermined range of percentiles comprised between the 70th and 100th percentile within a set formed by the estimated characteristic stride quantities, ordered by increasing values and occurring during one of the sub-periods,
o Repeat the previous step for several sub-periods.

**2.** The equipment (10) according to the preceding claim, wherein the characteristic quantity of a stride is an average velocity of the stride.

**3.** The equipment (10) according to any of the preceding claims, wherein the predetermined range of percentiles consists of the 95th percentile.

**4.** The equipment (10) according to any of the preceding claims, wherein the inertial sensor (11) comprises an accelerometer and/or a gyrometer.

**5.** The equipment (10) according to any of the preceding claims, wherein the inertial sensor (11) is configured to be attached to the lower limb (1) of the pedestrian, for example at an ankle (3) of the pedestrian.

**6.** The equipment (10) according to any of the preceding claims, wherein the determination of a stride comprises the detection for a given moment of an acceleration of absolute value greater than a predetermined threshold, this moment defining the start of the stride and the end of a previous stride.

**7.** The equipment (10) according to any of the preceding claims, wherein the length of a stride is the length of the projection of a path of the stride on a horizontal plane, or is the curvilinear length of a path of the stride, said path being estimated based on the motion measurements.

**8.** The equipment (10) according to claim 6 as dependent on claim 2, wherein the average velocity of a stride is a ratio between the length of the stride and the duration elapsed between its start and its end.

**9.** The equipment (10) according to any of the preceding claims, wherein the data processing unit (12) is further configured to verify a criterion of belonging of a stride to a continuous sequence of several consecutive strides, and to implement the steps of estimation, selection and repetition only on the strides belonging to a continuous sequence of several consecutive strides.

**10.** The equipment (10) according to claim 9, wherein a stride belongs to a continuous sequence of several consecutive strides when the duration between the start of said stride and the start of a previous or next stride is comprised between 0 and 10 seconds, preferably less than 3 seconds, and when said stride is part of a sequence of at least two consecutive strides, preferably at least six consecutive strides.

**11.** The equipment (10) according to any of the preceding claims, wherein a recording sub-period has a duration greater than 2 days, preferably greater than 15 days and/or a cumulative recording duration greater than 5 hours, 10 hours or 50 hours, preferably greater than 180 hours.

**12.** A computer program product comprising code instructions for the execution, when said program is executed on a computer, of a method for analyzing the stride of a walking pedestrian comprising steps of:

(a) Receiving measurements of a motion of a lower limb (1) of the pedestrian acquired during a recording period,
(b) Determining, by a data processing unit (12), each stride made by the pedestrian during the recording period, based on the motion measurements,
(c) Dividing the recording period into recording sub-periods,
(d) For several detected strides, estimating a characteristic quantity of the stride, based on the motion measurements, in which the characteristic quantity is or depends on a stride length,
(e) Selecting at least one characteristic stride quantity in a predetermined range of percentiles within a set formed by the estimated characteristic quantities, ordered by increasing values and occurring during a recording sub-period, the predetermined range of percentiles being comprised between the 70th and 100th percentile,
(f) Repeating step (e) for several recording sub-periods.

# FIG. 1

Foulée n    Foulée n+1

# FIG. 2

# FIG. 3

## FIG. 4

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- US 2013123665 A1

- FR 3042266

**Littérature non-brevet citée dans la description**

- **SEFERIAN A et al.** Longitudinal results of magneto-inertial motion analysis in Duchenne muscular dystrophy ambulant patients. *NEUROMUSCULAR DISORDERS*, 2016, vol. 26, ISSN 0960-8966

- **FOXLIN** ; **ERIC**. Pedestrian Tracking with Shoe-Mounted Inertial Sensors. IEEE Computer Society, November 2005, 38-46